# EUROPEAN PATENT APPLICATION

(11) **EP 3 711 666 A1**
(43) Date of publication of application: **23.09.2020**
(21) Application number: 19164402.0
(22) Date of filing: 21.03.2019
(51) Int. Cl.: A61B 5/103, A61B 5/11, A61B 5/00, G01C 5/00, G01C 22/00

(54) **ANALYSING MOVEMENT OF A SUBJECT**

(71) Applicant: Koninklijke Philips N.V., 5656 AG Eindhoven (NL)
(72) Inventor: SAPORITO, Salvatore, 5656 AE Eindhoven (NL); RISPENS, Sietse Menno, 5656 AE Eindhoven (NL)
(74) Representative: de Haan, Poul Erik

(57) **Abstract**

According to an aspect, there is provided a computer-implemented method for analysing movement of a subject. The method comprises obtaining, from a movement sensor in a device that is carried or worn by the subject, a movement signal representing movement of the subject during at least a first time period; obtaining, from an air pressure sensor in the device, an air pressure signal representing air pressure at the air pressure sensor during at least the first time period; processing the movement signal to identify a plurality of occurrences of a gait phase corresponding to the subject walking; for each of the identified occurrences of the gait phase, selecting a respective part of the air pressure signal corresponding in time to said occurrence of the gait phase; for a plurality of pairs of identified occurrences of the gait phase, determining a change in altitude of the subject between the identified occurrences of the gait phase in each pair from the respective selected parts of the air pressure signal; and determining if the subject has traversed stairs from the determined changes in altitude for the pairs of identified occurrences of the gait phase. A corresponding apparatus and computer program product are also provided.

## Description

### FIELD OF THE INVENTION

The invention relates to the analysis of movement of a subject, and in particular to a computer-implemented method, apparatus and computer program product for analysing movement of a subject to determine if the subject has traversed stairs.

### BACKGROUND OF THE INVENTION

Walking up and down stairs is a basic activity of daily living, and can become difficult or impossible for some people, particularly the elderly, or people with mobility issues.

Falling over is also an issue for elderly people, and falling can occur while traversing stairs (i.e. going up the stairs or going down the stairs) as a consequence of mobility and strength decline occurring due to the aging process or injuries, or reduced biomechanical and/or neuromuscular system responses. These falls, or fear of falling while traversing stairs, can result in altered or maladaptive movement patterns during stair traversal.

Many devices, such as physical activity monitors, are currently available that measure the movements of a person. These devices process the measurements to detect the footsteps of the person and detect when the person is walking. Various characteristics of a person's walking can be used by clinicians as a tool to assess the mobility of the person. In some cases, the characteristics can be used to assess a person's risk of falling.

Many of these devices have been developed to assess walking characteristics in a standardised setting, such as a healthcare clinic, but they therefore cannot provide an accurate reflection of the person's functional ability in their home environment or changes in the person's ability over time. Therefore some devices are being developed that can be worn or carried by the person in their home environment and that can provide an indication of when the person is walking and characteristics of the person's walking. Preferably these devices should be as unobtrusive as possible for the person, and thus some devices are provided so that they can be worn on the person's arm/wrist, worn at their waist or on their chest, or worn as a pendant around the person's neck. These devices can include a sensor, such as an accelerometer, to measure the movements of the person, and a sensor, such as an air pressure sensor, to provide measurements indicative of changes in altitude of the person, and the measurements are processed to identify when the person is walking. A device of this type is described in WO 2015/113915.

While a risk of falling or other mobility measure can be determined for a person based on their gait or walking/footstep pattern on a flat surface, it is considered that the person's gait or walking/footstep pattern while ascending or descending stairs may be more useful for evaluating, or more indicative of, fall risk or general mobility. Therefore, it is desirable to be able to automatically and reliably identify when the person has traversed stairs from the measurements. In addition, knowing whether a person can safely traverse stairs on their own (e.g. in their own home) is useful for assessing whether the person can continue to live independently.

However, even with both measurements of movements and altitude (or changes in altitude), reliable identification of stair traversal can be difficult. For example, a change in altitude occurring during walking may relate to stair traversal, or walking up or down a slope, walking around in a moving elevator, walking on uneven ground, etc. As another example, air pressure can vary for reasons other than changes in altitude, such as changes in the weather, or changes in the environment (e.g. a door or window being opened or closed, air conditioning being switched on or off, etc.). As another example, a person's walking/footstep pattern may have a high variability compared to someone else, or even compared to their previous stair traversals (e.g. they may be frail and have to take multiple footsteps per step/stair or rest between each step/stair). Another example is that there can be variability in the physical layout of the stairs being traversed relative to other stairs, e.g. differences in step height (also known as step rising), step depth (also known as step run), the presence/absence of treads, the tread materials, stringer inclination, whether the stairs are straight, whether the stairs are indoors or outdoors.

Therefore there is a need for improvements in the analysis of movement measurements and air pressure measurements to determine if the person has traversed stairs.

### SUMMARY OF THE INVENTION

According to a first specific aspect, there is provided a computer-implemented method for analysing movement of a subject, the method comprising obtaining, from a movement sensor in a device that is carried or worn by the subject, a movement signal representing movement of the subject during at least a first time period; obtaining, from an air pressure sensor in the device, an air pressure signal representing air pressure at the air pressure sensor during at least the first time period; processing the movement signal to identify a plurality of occurrences of a gait phase corresponding to the subject walking; for each of the identified occurrences of the gait phase, selecting a respective part of the air pressure signal corresponding in time to said occurrence of the gait phase; for a plurality of pairs of identified occurrences of the gait phase, determining a change in altitude of the subject between the identified occurrences of the gait phase in each pair from the respective selected parts of the air pressure signal; and determining if the subject has traversed stairs from the determined changes in altitude for the pairs of identified occurrences of the gait phase. Thus, the method provides improvements in the analysis of movement measurements and air pressure measurements to determine if the subject has traversed stairs by determining respective altitude changes between pairs of identified gait phases. This allows changes in altitude to be determined corresponding to a pair of gait phases occurring, regardless of their relative timing, which allows a more robust detection when the gait cycle is irregular, or when the time between consecutive gait cycles is so large that other non-altitude-related pressure changes can occur and affect the measured air pressure.

In some embodiments the step of determining if the subject has traversed stairs from the determined changes in altitude for the pairs of identified occurrences of the gait phase comprises: determining one or more statistics values from the determined changes in altitude for the pairs of identified occurrences of the gait phase; and determining if the subject has traversed stairs based on the determined one or more statistics values.

In some embodiments the step of determining if the subject has traversed stairs based on the determined one or more statistics values comprises comparing the one or more statistics values to one or more normal values and/or historical values for the subject when traversing stairs. These embodiments enable the method to be tailored to the subject and their stair traversing ability.

In either of the above embodiments, the step of determining if the subject has traversed stairs based on the determined one or more statistics values may comprise comparing the one or more statistics values to one or more standard values for a set of stairs. These embodiments enable altitude changes to be disregarded if they are not consistent with physical dimensions of a set of stairs.

In any of the above embodiments, the one or more statistics can comprise an average change in altitude per gait phase occurrence, an average change in altitude per gait phase occurrence for occurrences of the gait phase occurring in a predetermined time period, or a measure of variability of the determined changes in altitude for the pairs of identified occurrences of the gait phase.

In any of the above embodiments, the step of determining one or more statistics values can comprise forming a histogram from the determined changes in altitude for the pairs of identified occurrences of the gait phase; and the step of determining if the subject has traversed stairs comprises determining if the subject has traversed stairs based on the histogram.

In some embodiments the step of processing comprises processing the movement signal to identify at least three occurrences of the gait phase corresponding to the subject walking. These embodiments have the benefit that multiple pairs of the gait phase occurrences will be identified, and thus multiple altitude changes determined.

In some embodiments the gait phase is one of a heel strike, a mid-stance, a mid-swing, a toe-off and a foot-flat.

In some embodiments the step of selecting the respective part of the air pressure signal corresponding in time to said occurrence of the gait phase comprises: selecting a single measurement sample from the air pressure signal corresponding in time to the identified occurrence of the gait phase; or selecting a plurality of measurement samples from the air pressure signal corresponding in time to the identified occurrence of the gait phase.

In some embodiments, prior to the first time period, the method further comprises the steps of: obtaining one of a movement signal from the movement sensor for a second time period and an air pressure signal from the air pressure sensor for a second time period while the other one of the movement sensor and air pressure sensor is not active; processing the obtained one of the movement signal and the air pressure signal to determine a first value of a characteristic of the one of the movement signal and the air pressure signal; and if the determined first value meets a criterion, activating the other one of the movement sensor and air pressure sensor. These embodiments have the benefit that the power and/or resource consumption of the method is reduced when a stair traversal is unlikely to occur (e.g. when the amount of movement is low).

In these embodiments the method can further comprise the steps of obtaining a further one of the movement signal from the movement sensor for a third time period and an air pressure signal from the air pressure sensor for a third time period; processing the obtained further one of the movement signal and the air pressure signal to determine a second value of the characteristic; and if the determined second value does not meet the criterion, deactivating the other one of the movement sensor and air pressure sensor. These embodiments have the benefit of reducing the power and/or resource consumption of the method when a stair traversal is less unlikely to occur (e.g. once the amount of movement is below a threshold).

In these embodiments the characteristic may be one of a variance of the signal, a maximum amplitude of the signal, a minimum amplitude of the signal, a difference between a maximum amplitude and a minimum amplitude of the signal.

According to a second aspect, there is provided a computer program product comprising a computer readable medium having computer readable code embodied therein, the computer readable code being configured such that, on execution by a suitable computer or processor, the computer or processor is caused to perform the method of any of the first aspect or any embodiment thereof.

According to a third aspect, there is provided an apparatus for analysing movement of a subject, the apparatus comprising a processing unit configured to obtain, from a movement sensor in a device that is carried or worn by the subject, a movement signal representing movement of the subject during at least a first time period; obtain, from an air pressure sensor in the device, an air pressure signal representing air pressure at the air pressure sensor during at least the first time period; process the movement signal to identify a plurality of occurrences of a gait phase corresponding to the subject walking; for each of the identified occurrences of the gait phase, select a respective part of the air pressure signal corresponding in time to said occurrence of the gait phase; for a plurality of pairs of identified occurrences of the gait phase, determine a change in altitude of the subject between the identified occurrences of the gait phase in each pair from the respective selected parts of the air pressure signal; and determine if the subject has traversed stairs from the determined changes in altitude for the pairs of identified occurrences of the gait phase. Thus, the apparatus provides improvements in the analysis of movement measurements and air pressure measurements to determine if the subject has traversed stairs by determining respective altitude changes between pairs of identified gait phases. This allows changes in altitude to be determined corresponding to a pair of gait phases occurring, regardless of their relative timing, which allows a more robust detection when the gait cycle is irregular, or when the time between consecutive gait cycles is so large that other non-altitude-related pressure changes can occur and affect the measured air pressure.

In some embodiments the processing unit is configured to determine if the subject has traversed stairs from the determined changes in altitude for the pairs of identified occurrences of the gait phase by: determining one or more statistics values from the determined changes in altitude for the pairs of identified occurrences of the gait phase; and determining if the subject has traversed stairs based on the determined one or more statistics values.

In some embodiments the processing unit is configured to determine if the subject has traversed stairs based on the determined one or more statistics values by comparing the one or more statistics values to one or more normal values and/or historical values for the subject when traversing stairs. These embodiments enable the apparatus to tailor the stair traversal detection to the subject and their stair traversing ability.

In either of the above embodiments, the processing unit is configured to determine if the subject has traversed stairs based on the determined one or more statistics values by comparing the one or more statistics values to one or more standard values for a set of stairs. These embodiments enable altitude changes to be disregarded if they are not consistent with physical dimensions of a set of stairs.

In any of the above embodiments, the one or more statistics can comprise an average change in altitude per gait phase occurrence, an average change in altitude per gait phase occurrence for occurrences of the gait phase occurring in a predetermined time period, or a measure of variability of the determined changes in altitude for the pairs of identified occurrences of the gait phase.

In any of the above embodiments, the processing unit is configured to determine one or more statistics values by forming a histogram from the determined changes in altitude for the pairs of identified occurrences of the gait phase; and the processing unit is configured to determine if the subject has traversed stairs based on the histogram.

In some embodiments the processing unit is configured to process the movement signal to identify at least three occurrences of the gait phase corresponding to the subject walking. These embodiments have the benefit that multiple pairs of the gait phase occurrences will be identified, and thus multiple altitude changes determined.

In some embodiments the gait phase is one of a heel strike, a mid-stance, a mid-swing, a toe-off and a foot-flat.

In some embodiments the processing unit is configured to select the respective part of the air pressure signal corresponding in time to said occurrence of the gait phase by: selecting a single measurement sample from the air pressure signal corresponding in time to the identified occurrence of the gait phase; or selecting a plurality of measurement samples from the air pressure signal corresponding in time to the identified occurrence of the gait phase.

In some embodiments, the processing unit is further configured to, prior to the first time period, obtain one of a movement signal from the movement sensor for a second time period and an air pressure signal from the air pressure sensor for a second time period while the other one of the movement sensor and air pressure sensor is not active; process the obtained one of the movement signal and the air pressure signal to determine a first value of a characteristic of the one of the movement signal and the air pressure signal; and activate the other one of the movement sensor and air pressure sensor if the determined first value meets a criterion. These embodiments have the benefit that the power and/or resource consumption of the processing unit/apparatus is reduced when a stair traversal is unlikely to occur (e.g. when the amount of movement is low).

In these embodiments the processing unit can be further configured to obtain a further one of the movement signal from the movement sensor for a third time period and an air pressure signal from the air pressure sensor for a third time period; process the obtained further one of the movement signal and the air pressure signal to determine a second value of the characteristic; and deactivate the other one of the movement sensor and air pressure sensor if the determined second value does not meet the criterion. These embodiments have the benefit of reducing the power and/or resource consumption of the processing unit/apparatus when a stair traversal is less unlikely to occur (e.g. once the amount of movement is below a threshold).

In these embodiments the characteristic may be one of a variance of the signal, a maximum amplitude of the signal, a minimum amplitude of the signal, a difference between a maximum amplitude and a minimum amplitude of the signal.

According to a fourth aspect, there is provided a system for analysing movement of a subject, The system comprises a device that is to be carried or worn by the subject, the device comprising a movement sensor for measuring movement of the subject and outputting a movement signal representing movement of the subject and an air pressure sensor for measuring air pressure and outputting an air pressure signal representing air pressure at the air pressure sensor; and an apparatus according to the third aspect or any embodiment thereof.

In some embodiments the apparatus is part of the device. In alternative embodiments the apparatus is separate from the device.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a block diagram illustrating a system comprising an apparatus and device according to an exemplary embodiment;
Fig. 2 is a flow chart illustrating a method according to an exemplary embodiment; and
Figs. 3-7 show respective sets of graphs showing movement measurements, altitude changes and a histogram of altitude changes between heel strikes for subjects in different mobility scenarios.

### DETAILED DESCRIPTION OF EMBODIMENTS

As noted above, it is desirable to provide improvements in the analysis of movement measurements and air pressure measurements to determine if a person (referred hereafter as a 'subject') has traversed stairs. As used herein, 'stairs' or a 'set of stairs' refers to an arrangement that divides a large vertical distance into a series of smaller changes in vertical distance (each referred to as a 'step' or 'stair') that can be traversed by a subject in turn to enable the subject to move from a lower altitude to a higher altitude, and vice versa. A set of stairs, as referred to herein, comprises at least two steps, or, in some cases, at least three steps. The term 'traversing stairs' as used herein refers to the subject ascending (walking up) a set of stairs or descending (walking down) a set of stairs, and 'traversed stairs' as used herein refers to the subject having ascended (walked up) at least a plurality (or at least three, or all) steps of a set of stairs or having descended (walked down) at least a plurality (or at least three, or all) steps of a set of stairs. A set of stairs can also include one or more 'landings', which are flat areas between consecutive steps/stairs on which a subject may have to turn (e.g. 90° or 180) before traversing the next steps/stairs. It is an aim of the analysis described herein to distinguish between walking up or down a set of stairs and walking along a flat surface, walking on uneven terrain, and/or to reduce false positive detections of stair traversal in the event that air pressure changes due to reasons other than the altitude changing.

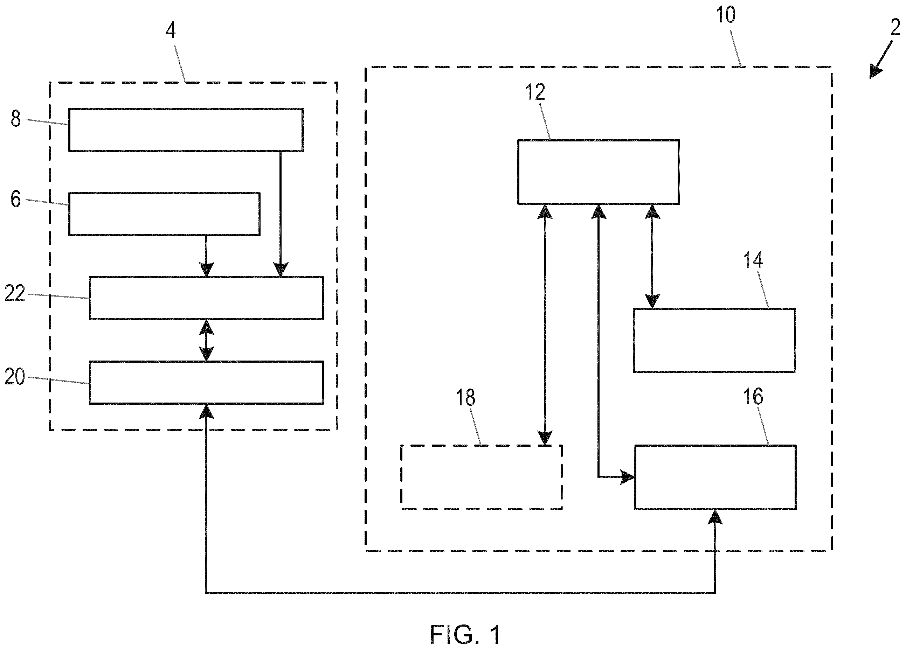

Fig. 1 illustrates a system 2 according to an exemplary embodiment of the teachings presented herein. In this embodiment the system 2 comprises a device 4 that is carried or worn by the subject and that includes a movement sensor 6 that is provided to measure the movements of the subject and an air pressure sensor 8 that is provided to measure the air pressure in the environment around or at the subject. The system 2 also comprises an apparatus 10 that receives the movement measurements and the air pressure measurements from the device 4 and analyses the measurements to determine if the subject has traversed stairs.

The device 4 can be in any form suitable to enable the subject to carry or wear the device 4. For example, the device 4 may be in the form of a watch or smartwatch, a smartphone, a bracelet, a pendant, a necklace, a chest band, integrated into an item of clothing, etc. In some embodiments, as shown in Fig. 1, the apparatus 10 can be separate from the device 4. In these embodiments, the apparatus 10 can be any type of electronic device or computing device that can communicate with, or otherwise receive the movement measurements and air pressure measurements directly or indirectly from, the device 4. For example the apparatus 10 can be, or be part of, a computer, a laptop, a tablet, a smartphone, a smartwatch, etc., and as such may be an apparatus that is present or used in the home or care environment of the subject. In other implementations, the apparatus 10 can be an apparatus that is remote from the subject, and remote from the home or care environment of the subject. For example, the apparatus 10 can be a server, for example a server in a data centre (also referred to as being 'in the cloud'). In alternative embodiments, the apparatus 10 (and in particular the functionality of the apparatus 10 as described herein) can be integral with the device 4. Therefore the apparatus 10 can also be carried or worn by the subject as part of the device 4.

The movement sensor 6 can include any type of sensor(s) for measuring the movements of a subject, or for providing measurements representative of the movements of a subject. The movement sensor 6 generates and outputs a movement signal representing measurements of movement of the subject. The movement signal can comprise a time series of movement measurements (samples), and the movement signal can therefore relate to the movements in a time period. The movement sensor 6 can use any desired sampling frequency, for example 50 measurements per second (50 Hz), 60 Hz or 100 Hz. The accelerometer can generate and output a movement signal that contains a plurality of acceleration measurement samples representing the movements of the subject at a plurality of time instants. The accelerometer is typically an accelerometer that measures accelerations in three dimensions, and the movement signal generated by the accelerometer 108 can include respective measurements representing the accelerations in each of the three dimensions. For example, the accelerometer can output respective measurement signals for each of an x-axis, y-axis and z-axis of a Cartesian coordinate system. In alternative embodiments, the movement sensor 6 can include, or further include, any one or more of a magnetometer, a satellite positioning system receiver (e.g. a GPS receiver, a GLONASS receiver, a Galileo positioning system receiver), a gyroscope, or a pressor sensor that can be positioned in the subject's shoe (or in each shoe) or other footwear to measure the pressure that the foot is applying to the ground (since these measurements can be indicative of footsteps).

The air pressure sensor 8 can include any type of sensor for measuring air pressure or changes in air pressure. The air pressure sensor 8 generates and outputs an air pressure signal representing measurements of air pressure or changes in air pressure at the air pressure sensor 8. The air pressure signal can comprise a time series of air pressure measurements (samples) and the air pressure signal can therefore relate to the air pressure or changes in air pressure in a time period. The air pressure sensor 8 can use any desired sampling frequency, for example 1 Hz or 50 Hz.

The apparatus 10 includes a processing unit 12 that controls the operation of the apparatus 10 and that can be configured to execute or perform the methods described herein. In particular, the processing unit 12 can obtain the movement signal/movement measurements and the air pressure signal/air pressure measurements and processing them to determine if the subject has traversed stairs. The processing unit 12 can be implemented in numerous ways, with software and/or hardware, to perform the various functions described herein. The processing unit 12 may comprise one or more microprocessors or digital signal processor (DSPs) that may be programmed using software or computer program code to perform the required functions and/or to control components of the processing unit 12 to effect the required functions. The processing unit 12 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The processing unit 12 is connected to a memory unit 14 that can store data, information and/or signals (including movement measurements and/or air pressure measurements) for use by the processing unit 12 in controlling the operation of the apparatus 10 and/or in executing or performing the methods described herein. In some implementations the memory unit 14 stores computer-readable code that can be executed by the processing unit 12 so that the processing unit 12 performs one or more functions, including the methods described herein. In particular embodiments, the program code can be in the form of an application for a smartwatch, a smartphone, tablet, laptop or computer. The memory unit 14 can comprise any type of non-transitory machine-readable medium, such as cache or system memory including volatile and non-volatile computer memory such as random access memory (RAM) static RAM (SRAM), dynamic RAM (DRAM), read-only memory (ROM), programmable ROM (PROM), erasable PROM (EPROM) and electrically erasable PROM (EEPROM), implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

In the embodiment of the system 2 shown in Fig. 1, as the apparatus 10 is separate from the device 4 that includes the movement sensor 6 and air pressure sensor 8, the apparatus 10 also includes interface circuitry 16 for enabling a data connection to and/or data exchange with other devices, including device 4, and optionally any one or more of servers, databases, user devices, and sensors. The connection may be direct or indirect (e.g. via the Internet), and thus the interface circuitry 16 can enable a connection between the apparatus 10 and a network, such as the Internet, or between the apparatus 10 and device 4, via any desirable wired or wireless communication protocol. For example, the interface circuitry 16 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). In the case of a wireless connection, the interface circuitry 16 (and thus apparatus 10) may include one or more suitable antennas for transmitting/receiving over a transmission medium (e.g. the air). Alternatively, in the case of a wireless connection, the interface circuitry 16 may include means (e.g. a connector or plug) to enable the interface circuitry 16 to be connected to one or more suitable antennas external to the apparatus 10 for transmitting/receiving over a transmission medium (e.g. the air). The interface circuitry 16 is connected to the processing unit 12 to enable information or data received by the interface circuitry 16 to be provided to the processing unit 12, and/or information or data from the processing unit 12 to be transmitted by the interface circuitry 16.

The interface circuitry 16 can be used to receive movement measurements generated by the movement sensor 6 and air pressure measurements generated by the air pressure sensor 8.

In some embodiments, the interface circuitry 16 can be used to output a result of the processing by the processing unit 12, for example an indication of whether the subject has traversed stairs, and/or information relating the stair traversal by the subject.

In some embodiments, the apparatus 10 comprises a user interface 18 that includes one or more components that enables a user of apparatus 10 (e.g. the subject, or a care provider for the subject) to input information, data and/or commands into the apparatus 10 (e.g. for starting or enabling the analysis of movement measurements and air pressure measurements according to the techniques described herein), and/or enables the apparatus 10 to output information or data to the user of the apparatus 10. An output may be an audible, visible and/or tactile indication that the subject has traversed stairs, for example. The user interface 18 can comprise any suitable input component(s), including but not limited to a keyboard, keypad, one or more buttons, switches or dials, a mouse, a track pad, a touchscreen, a stylus, a camera, a microphone, etc., and the user interface 18 can comprise any suitable output component(s), including but not limited to a display screen, one or more lights or light elements, one or more loudspeakers, a vibrating element, etc.

It will be appreciated that a practical implementation of apparatus 10 may include additional components to those shown in Fig. 1. For example the apparatus 10 may also include a power supply, such as a battery, or components for enabling the apparatus 10 to be connected to a mains power supply.

As noted above, the movement sensor 6 and air pressure sensor 8 are part of device 4, which is separate from the apparatus 10 in the embodiment shown in Fig. 1. In order for the movement measurements and the air pressure measurements to be communicated from the device 4 to the apparatus 10, the device 4 comprises interface circuitry 20. The interface circuitry 20 may be implemented in a similar way to the interface circuitry 16 in the apparatus 10.

In some embodiments, the device 4 can also include a processing unit 22 for controlling the operation of the device 4. This processing unit 22 can also be used to perform some pre-processing of the movement measurements and/or air pressure measurements before they are communicated to the apparatus 10, for example the measurements can be filtered to reduce or remove a noise component or artefacts. The processing unit 22 may be implemented in a similar way to the processing unit 12 in the apparatus 10.

It will be appreciated that a practical implementation of device 4 may include additional components to those shown in Fig. 1. For example the device 4 may also include a power supply, preferably a battery so that the device 4 is portable, or components for enabling the device 4 to be connected to a mains power supply.

In alternative embodiments of the system 2 where the apparatus 10 is part of the device 4, it will be appreciated that only one processing unit 12/22 may be present, and interface circuitry is not required to communicate the movement measurements and air pressure measurements to the processing unit 12.

Conventional techniques typically attempt to detect stair traversal by considering an altitude change over a number of footsteps (e.g. a 10-second period, or over a complete walking activity). According to the techniques described herein, altitude changes are considered for a specific gait phase (e.g. a heel strike), instead of only the total change in altitude during an entire walk (multiple steps). This enables the detection that the subject has traversed stairs to be improved, particularly for elderly or frail subjects who typically have a fragmented walking behaviour when traversing stairs, for example with a shorter series of steps and/or with frequent interruptions/rest periods. When the traversal of stairs is fragmented, the assumption of the subject traversing the stairs with a constant walking speed (as in conventional techniques where a total change in altitude can be divided by a number of steps, or a total change in altitude can be divided by total time taken) is not valid, and so the accuracy of the conventional techniques can be limited by spurious changes in altitude caused by other (non-altitude-related) changes in the air pressure measurements, which accumulate and lead to altitude change estimates that are not representative of the actual change in altitude during the entire walking event, and thus, to unrealistic values for average change in altitude per step.

In contrast, the techniques described herein provide improvements in the analysis of movement measurements and air pressure measurements to determine if the subject has traversed stairs by identifying occurrences of a gait phase (e.g. heel strike) in the movement measurements, determining a respective altitude change between pairs of identified gait phases from the air pressure measurements, and determining if the subject has traversed stairs from the determined changes in altitude. Thus, the techniques described herein provide that changes in altitude between footsteps can be determined, regardless of their relative timing, which allows a more robust detection when the footsteps are irregular, or when the time between consecutive footsteps is so large that other non-altitude-related pressure changes can occur and affect the measured air pressure. For example observing altitude changes at a gait phase level rather than a total change in altitude over a longer walking period means that changes in air pressure due to, for example, changes in the environment (e.g. a door or window opening or closing) which manifests as a relatively sudden and large change in air pressure (compared to an air pressure change expected to occur for ascending or descending a single step) will not lead directly to a detection that the subject has traversed stairs.

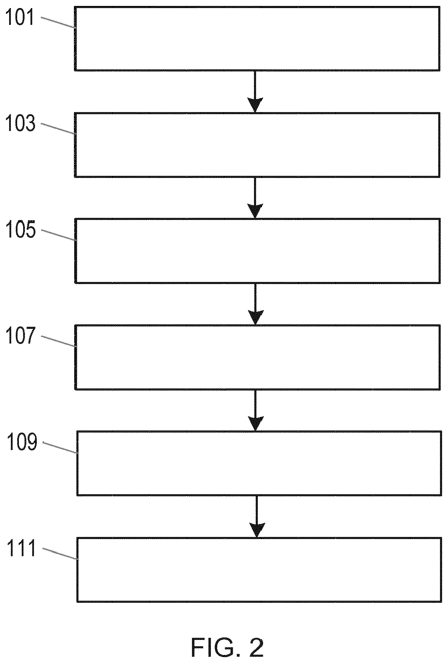

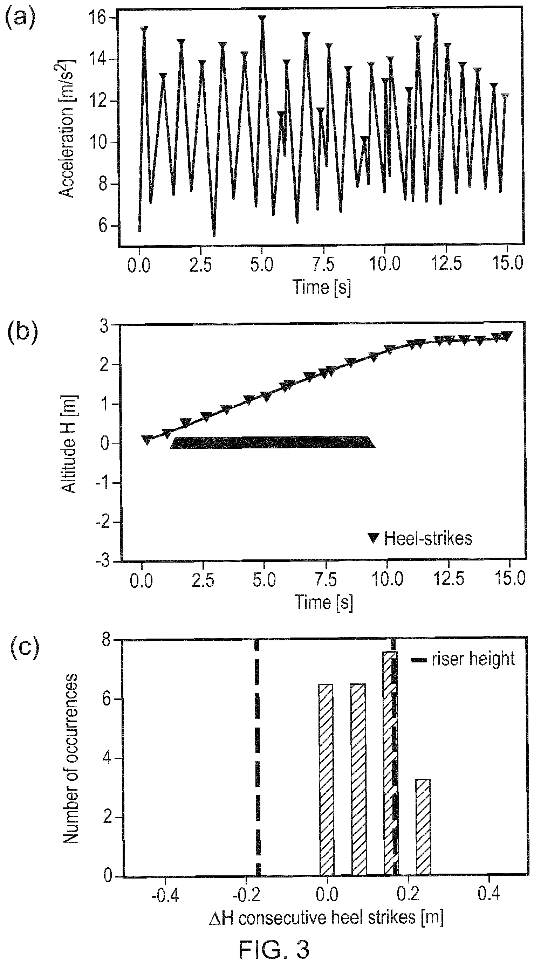

The flow chart in Fig. 2 illustrates an exemplary method according to the techniques described herein. One or more of the steps of the method can be performed by the processing unit 12 in the apparatus 10, in conjunction with any of the memory unit 14, interface circuitry 16 and user interface 18 as appropriate. The processing unit 12 may perform the one or more steps in response to executing computer program code, that can be stored on a computer readable medium, such as, for example, the memory unit 14. The flow chart in Fig. 2 is described with reference to the examples shown in Figs. 3-7, which show graphs of movement measurements and air pressure measurements obtained for different scenarios and histograms formed according to particular embodiments of the method.

In a first step, step 101, the processing unit 12 obtains a movement signal representing movement of the subject during at least a first time period. The movement signal is obtained from movement sensor 6 in device 4. The device 4 is being carried or worn by the subject at least during the first time period. The processing unit 12 can obtain the movement signal directly from the movement sensor 6 or indirectly from the movement sensor 6 (e.g. via interface circuitry 16 and interface circuitry 20). In these embodiments the processing unit 12 may be able to process the measurements as they are received (e.g. in real-time or near-real-time) to determine if the subject has traversed stairs (and may still be traversing stairs). Alternatively, the processing unit 12 can retrieve the movement signal from the memory unit 14. In some embodiments the processing unit 12 can receive the movement signal representing movement of the subject during the first time period after the first time period has passed. Alternatively, the processing unit 12 can receive the movement signal over the course of the first time period as the movement of the subject is measured. The first time period preferably has a duration that is long enough to cover a typical stair traversal activity by the subject. A set of stairs in a house may typically include 5-15 steps. In some embodiments, the first time period can be at least 5 seconds (s), at least 10s, at least 20s, or at least 1 minute.

Fig. 3(a) shows an exemplary movement (acceleration) signal for a 15-second time period. The measurements shown in Fig. 3 were obtained from a device 4 worn or carried by a subject that walked up a set of stairs during part of the 15-second time period.

In a second step, step 103 (which can occur before, after or at the same time as step 101), the processing unit 12 obtains an air pressure signal representing air pressure at the air pressure sensor 8 during at least the first time period. As movement measurements are also obtained for at least the first time period, movement measurements and air pressure measurements for the first time period are both available to the processing unit 12 for analysis. The air pressure signal is obtained from air pressure sensor 8 in device 4 that is being carried or worn by the subject at least during the first time period, and therefore the air pressure measurements represent the air pressure in the environment around or at the subject. The processing unit 12 can obtain the air pressure signal directly from the air pressure sensor 8 or indirectly from the air pressure sensor 8 (e.g. via interface circuitry 16 and interface circuitry 20). In these embodiments the processing unit 12 may be able to process the measurements as they are received (e.g. in real-time or near-real-time) to determine if the subject has traversed stairs (and may still be traversing stairs). Alternatively, the processing unit 12 can retrieve the air pressure signal from the memory unit 14. In some embodiments the processing unit 12 can receive the air pressure signal representing movement of the subject during the first time period after the first time period has passed. Alternatively, the processing unit 12 can receive the air pressure signal over the course of the first time period as the air pressure at the subject is measured.

In step 105, the processing unit 12 processes the movement signal to identify a plurality of occurrences of a gait phase corresponding to the subject walking. As is known, the process of walking is a cyclic (i.e. repeating) series of 'gait phases', with each walking 'cycle' (referred to herein as a 'gait cycle') including, gait phases corresponding to a heel strike (where the heel of the foot strikes or impacts the ground), a stance phase where the foot is on the ground (referred to as 'foot-flat' or 'mid-stance') and provides support for the body weight while the other foot is off the ground and moving forward, a swing phase where the foot is lifted off the ground (referred to as 'toe-off) and moved forward (with the point of maximum forward velocity being referred to as 'mid-swing') and placed on the ground (heel-strike). It will be appreciated that a single gait cycle will include two occurrences of each of these gait phases, e.g. each gait cycle will include two heel strikes, one by the left foot and one by the right foot.

Thus, in some embodiments of step 105, the processing unit 12 can process the movement measurements to identify a series of impacts due to heel strikes (e.g. in the case of acceleration measurements, a magnitude of acceleration greater than a threshold, or in the case of a foot pressure sensor, an increase in the measured pressure to above a threshold), a series of static stance gait phases (e.g. zero-crossings of a derivative of the measured acceleration with respect to time), a series of toe-off points (e.g. where there is acceleration in an upward direction, or in the case of a foot pressure sensor, where the measured pressure falls below a or the threshold), and/or a series of mid-swing points (e.g. where velocity of a foot or leg in a forward direction exceeds a threshold).

It will be appreciated that the type of gait phase that the processing unit 12 can identify (or reliably identify) in the movement measurements can depend on the type of movement sensor 6 in the device 4 and/or on where on the body of the subject the device 4 is located. For example, a mid-swing gait phase or toe-off point might be easier to identify where the movement sensor 6 is worn or carried on a leg or foot, and a heel-strike may be easier to identify in acceleration measurements as opposed to, say, gyroscope measurements.

In some embodiments, in step 105 the processing unit 12 is required to identify at least three occurrences of the gait phase before 'walking' can be detected. Typically, where the subject was walking during the first time period (including walking up or down stairs), the processing unit 12 will be able to identify many more than three occurrences of the gait phase.

Various techniques for processing movement measurements (including acceleration measurements) to identify if a subject is walking are known to those skilled in the art, and various such techniques can be used in step 105 to identify a plurality of occurrences of the gait phase corresponding to the subject walking. One exemplary technique is described in WO 2015/113915, while another exemplary technique is described in WO 2011/004322.

The example in Fig. 3(a) is an acceleration signal, and the processing unit 12 can analyse this signal to identify the heel-strike gait phase as, for example, the time where maximum acceleration occurs in any peak in the acceleration signal that is above a threshold. A series of heel-strikes, which correspond to alternating heel strikes by the left foot and the right foot, are marked in Fig. 3(a) by inverted triangles.

Next, in step 107, for each of the identified occurrences of the gait phase (e.g. each identified heel strike), the processing unit 12 selects a respective part of the air pressure signal corresponding in time to the identified occurrence of the gait phase. The part of the air pressure signal selected for each of the identified occurrences of the gait phase in step 107 is used as the air pressure for that gait phase occurrence in subsequent steps of the method. As the selected parts of the air pressure signal corresponds in time to the same type of gait phase in each gait cycle, the selected parts of the air pressure signal for the occurrences of the gait phase will be more directly comparable to each other. For example, the part of the air pressure signal selected could correspond in time to each heel strike identified in the movement measurements. The use of the heel strike as the gait phase identified in the movement measurements has an advantage over the use of the mid-swing phase or mid-stance phase as the ascending or descending of a step/stair occurs during the mid-swing phase for one foot and the mid-stance phase for the other foot, and therefore the use of a part of the air pressure signal corresponding in time to the mid-swing phase or the mid-stance phase could provide a misleading or less reliable air pressure value/altitude change. As another example, the part of the air pressure signal selected for each identified gait phase occurrence could correspond in time to the median time between each consecutive pair of detected heel strikes.

In some embodiments, in step 107 a single measurement sample is selected from the air pressure signal. For example, the air pressure measurement sample corresponding in time to the timing of an identified heel strike can be selected. In alternative embodiments, in step 107 multiple consecutive measurement samples can be selected from the air pressure signal. The multiple samples can start or end at the time of the relevant gait phase occurrence, or the time period covered by the multiple consecutive measurement samples can span the time of the relevant gait phase occurrence. For example, step 107 can comprise selecting a plurality of measurement samples (e.g. 5) before the time of the relevant gait phase occurrence and a plurality of measurement samples (e.g. 5) after the time of the relevant gait phase occurrence. In some embodiments, the plurality of measurement samples can be averaged (e.g. as a median, mode or mean) or otherwise combined to provide a single measurement sample representing the air pressure for that gait phase occurrence. It will be appreciated that where a plurality of measurement samples are selected in step 107, the measurement samples should all relate to the same gait phase occurrence, i.e. none of the measurement samples selected should relate to a neighbouring gait phase occurrence.

It will be appreciated that the selecting in step 107 can be understood as 'subsampling' the air pressure signal, as only parts of the air pressure signal are used in the subsequent analysis of the movements of the subject.

Next, in step 109, for pairs of identified occurrences of the gait phase, a change in altitude of the subject between the identified occurrences of the gait phase in each pair is determined. This change in altitude is determined from the respective parts of the air pressure signal selected in step 107 for those gait phase occurrences, and the change in altitude can be given by the difference in the values of the selected air pressure measurement samples in step 107, with that difference converted into altitude or a change in altitude using a conventional relationship between air pressure and altitude. Alternatively, the air pressure measurement samples selected in step 107 for the occurrences of the gait phase can be converted into altitude (a relative altitude or absolute altitude) using a conventional relationship between air pressure and altitude, and the change in altitude can be determined as the difference between the two altitudes. For example in step 109 the air pressure measurement samples corresponding to two heel strikes can be compared to determine the change in altitude of the subject between the two heel-strikes.

In some embodiments of step 109, the pairs of identified occurrences are consecutive occurrences of the identified gait phase. For example, each pair can be consecutive footsteps (e.g. a left foot step and a right foot step), and the change in altitude can be determined from the respective parts of the air pressure signal for those footsteps.

In other embodiments of step 109, the pairs of identified occurrences are non-consecutive occurrences of the gait phase. In some embodiments, the non-consecutive occurrences of the gait phase in the pair can be occurrences of the gait phase that have one other (or a plurality of) occurrence of the gait phase between them. For example, each pair can be consecutive footsteps with a particular foot (e.g. a pair of consecutive steps with the left foot (in which case there is a right foot step in between them that is not part of the pair)). As another example, each pair can be footsteps that have 9 other occurrences of an identified footstep between them (e.g. one of the footsteps is the tenth footstep after the other). In either case, the change in altitude can be determined from the respective parts of the air pressure signal for those non-consecutive footsteps.

Some embodiments of step 109 can determine the change in altitude using several of the types of pairs of occurrences set out above (e.g. pairs comprising consecutive identified occurrences of the gait phase, and pairs comprising non-consecutive identified occurrences of the gait phase). In this case, it will be appreciated that some conversion or scaling of the determined changes in altitude may be required in order for the changes in altitude for the pairs covering different numbers of gait cycles to be compared. For example, a pair corresponding to a left foot step and the next left foot step is one gait cycle and so the change in altitude represents the change over one gait cycle, whereas a pair corresponding to a left foot step and the fifth left foot step after that is five gait cycles and so the change in altitude represents the change over five gait cycles, and so one of these changes in altitude needs to be scaled to be comparable to the other.

Fig. 3(b) shows an exemplary altitude signal for the same 15-second time period represented in Fig. 3(a) where the subject is ascending stairs. This altitude signal (with altitude given in metres, m) can be derived from the air pressure signal obtained in step 103 using a conventional relationship between air pressure and altitude, with the initial altitude (i.e. at the start of the 15-second time period) being set to 0. It can be seen that the altitude increases generally linearly for the first 10 seconds, and is relatively constant for the remaining 5 seconds. Fig. 3(b) shows the parts of the air pressure signal selected in step 107 (albeit converted to altitude) for each of the identified occurrences of the gait phase (and specifically the altitude at each of the detected heel strikes). According to embodiments of step 109, the change in altitude between each consecutive pair of occurrences of the gait phase can be determined as the difference between the altitudes for each consecutive pair of heel strikes.

Step 109 will generate respective changes in altitude for a plurality of pairs of occurrences of the gait phase, and in step 111 these changes in altitude are analysed to determine if the subject has traversed stairs.

In particular embodiments of step 111, the processing unit 12 determines one or more statistics values from the determined changes in altitude for the pairs of occurrences of the gait phase. The one or more statistics can comprise an average change in altitude per gait phase occurrence (so an average (e.g. median, mode or mean) of the changes in altitude determined in step 109), an average change in altitude per gait phase occurrence for gait cycles occurring in a predetermined time period (e.g. an average of the altitude changes for a subset or respective subsets of the identified gait cycles), a measure of variability of the determined changes in altitude across the pairs of identified occurrences of the gait phase (for example the standard deviation of the determined changes in altitude, or the inter-quartile range) and/or a measure of variability of the determined changes in altitude for the occurrences of the gait phase occurring in a predetermined time period (e.g. a variability measure of the altitude changes for a subset of the identified gait phase occurrences).

In addition, the statistics value determined in step 111 could also include respective measures of the variability (e.g. standard deviation) or the average (e.g. mean) of the changes in altitude for subsets of consecutive occurrences of the gait phase. For example each subset can include 10 consecutive occurrences of the gait phase. In some embodiments, the statistics value determined in step 111 could also include respective measures of the variability or the average of the changes in altitude for overlapping subsets of consecutive occurrences of the gait phase (e.g. 10 consecutive occurrences).

The determined statistics values can then be used to determine if the subject has traversed stairs during the first time period. For example the statistics values can be used to determine if the subject has traversed stairs by comparing the one or more statistics values to one or more normal values and/or historical values that correspond to values that might be or have been observed when the subject or a population of subjects is traversing stairs. If the derived statistics values are consistent with the normal values and/or historical values associated with stair traversal, then in step 111 it can be determined that the subject has traversed stairs. On the other hand, if the derived statistics values are not consistent with the normal values and/or historical values associated with stair traversal, then in step 111 it can be determined that the subject has not traversed stairs.

Alternatively or in addition, the statistics values can be used to determine if the subject has traversed stairs by comparing the one or more statistics values to one or more standard values for a set of stairs, for example a stair height (e.g. height between each step/stair in the set of stairs). If the derived statistics values are consistent with the standard values for a set of stairs, then in step 111 it can be determined that the subject has traversed stairs. On the other hand, if the derived statistics values are not consistent with the standard values for a set of stairs, then in step 111 it can be determined that the subject has not traversed stairs.

In a first example, for movement measurements and air pressure measurements obtained for a particular time period, the mean change in height per heel strike is +40±5 centimetres (cm). In step 111, the movement by the subject in this time period will not be classified as traversed stairs as 40cm is too large for a typical riser height (step height) in a set of stairs and/or is not consistent with the typical step height that can be traversed by the subject.

In a second example, for movement measurements and air pressure measurements obtained for a different time period, the mean change in height per heel strike is -15±2 cm. In step 111, the movement by the subject in this time period can be classified as descending stairs as a 15 cm altitude change is consistent with a typical riser height in a set of stairs.

However, in a third example, which is based on the same measurements as the second example, historical data for the subject is also available that indicates that the subject has only previously (or recently) been able to traverse stairs at a rate of one step/stair per full gait cycle (so the subject has to get both feet on to the same stair/step (two heel strikes) before stepping up to the next step/stair). In this case, an average altitude change of -15 cm per heel strike for the subject is not consistent with the historical data for the subject and so this walking by the subject will not be classified as a stair traversal.

In a fourth example, which is based on another set of measurements, a mean determined change in altitude per heel strike is -15±2 cm over 10 identified heel strikes. This change in altitude is compatible with the subject descending stairs, and the historical data of the subject indicates that the subject traverses a series of 10 steps/stairs each day, and so it can be identified as the subject having traversed (descended) stairs.

In some embodiments, further information or measurements can be used to improve the detection of whether the subject has traversed stairs. For example, in some embodiments, the temporal sequence of determined changes in altitude for the paired occurrences of the gait phase, including their order, can be analysed or used in any way to determine if the subject has traversed stairs. For example, blocks of 10 heel strikes with a relatively constant positive altitude change separated by 3 heel strikes without a change in altitude are compatible with the subject ascending a set of stairs that has a landing area halfway up on which the subject has to turn). More generally, statistical values (e.g. mean or median change in height per footstep) and also the sequence and the order of the changes in altitude may be used to determine whether the subject has traversed stairs or not.

As another or further example, location information for the subject can provide an indication of whether the subject is in an environment where stairs are present, and this can increase the likelihood that stair traversal is detected (and vice versa). Location information can be provided by a satellite positioning system receiver (e.g. GPS), and/or by other components of the device 4. For example, the interface circuitry 20 in the device 4 can scan for WiFi networks, and the identified network(s) can be used to determine the location of the subject. In the fourth example above, the likelihood of the subject being determined to have traversed stairs can be increased if the historical data indicates that the subject traverses the 10 steps/stairs each day in their home, and at the time that the 10 heel strikes are identified, the home WiFi network is visible to the interface circuitry 20 in the device 4.

In some embodiments, step 111 can comprise forming a histogram from the determined changes in altitude for the pairs of occurrences of the gait phase, with the histogram having bins for different ranges of changes in altitude (e.g. a bin for changes in altitude between -0.05 m and +0.05 m, a bin for changes in altitude between -0.15 m and - 0.05 m, etc.). The histogram can then be used or analysed to determine if the subject has traversed stairs. Fig. 3(c) shows a histogram formed from the change in altitude between consecutive heel strikes identified in the movement measurements, and the histogram also includes vertical bars at around +0.18 m and -0.18 m corresponding to the riser height of a step/stair. It can be seen that the histogram is generally centred on the riser height of +0.18 m, and so this movement can be classified as the subject traversing (ascending) stairs.

The histogram determined in step 111 can be analysed or used in any way to determine if the subject has traversed stairs. For example, the shape of the distribution represented in the histogram can be assessed using quantitative measures, such as central and/or non-central statistical moments of the histogram (e.g. mean, asymmetry, inter-quartile range, total sum of the change in altitude per step), and/or the temporal consistency of the determined changes in altitude and the gait phases can be assessed as mentioned above.

As before, the information determined from the histogram can be compared to one or more normal values and/or historical values that correspond to values that might be or have been observed when the subject or a population of subjects is traversing stairs. If the derived statistics values are consistent with the normal values and/or historical values associated with stair traversal, then in step 111 it can be determined that the subject has traversed stairs. On the other hand, if the derived statistics values are not consistent with the normal values and/or historical values associated with stair traversal, then in step 111 it can be determined that the subject has not traversed stairs. Likewise, as indicated above with reference to Fig. 3(c), the information determined from the histogram can be compared to one or more standard values for a set of stairs, for example a stair height (e.g. height between each step/stair in the set of stairs). If the derived statistics values are consistent with the standard values for a set of stairs, then in step 111 it can be determined that the subject has traversed stairs. On the other hand, if the derived statistics values are not consistent with the standard values for a set of stairs, then in step 111 it can be determined that the subject has not traversed stairs.

As noted above, Fig. 3 shows an exemplary movement (acceleration) signal for a 15-second time period, a corresponding exemplary air pressure signal, and a histogram formed from the changes in altitude between consecutive identified heel strikes, for a subject that is ascending stairs.

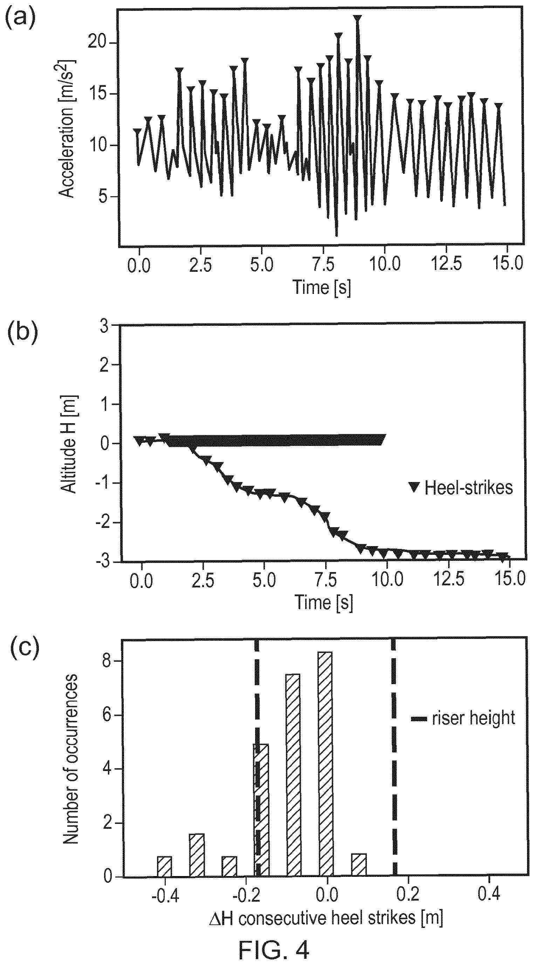

Figs. 4-7 show other exemplary movement scenarios for a subject where a histogram is formed from the changes in altitude between consecutive heel strikes identified in acceleration measurements. The acceleration measurements in each case were obtained for a 15-second time period by an accelerometer in a device 4 worn or carried by a subject. Likewise, the air pressure measurements were obtained by an air pressure sensor 8 in the device 4.

In Fig. 4, the measurements are for a subject that is descending stairs. Thus, Fig. 4(a) shows an exemplary acceleration signal for the 15-second time period, with a series of identified heel-strikes, which correspond to alternating heel strikes by the left foot and the right foot, marked by inverted triangles. Fig. 4(b) shows an exemplary altitude signal for the same 15-second time period represented in Fig. 4(a). This altitude signal is derived from the air pressure signal using a conventional relationship between air pressure and altitude with the initial altitude (i.e. at the start of the 15-second time period) being set to 0. It can be seen that the altitude decreases generally linearly for the first 10 seconds, and is relatively constant for the remaining 5 seconds. Fig. 4(b) shows the parts of the air pressure signal selected according to step 107 for each of the identified heel strikes. Fig. 4(c) shows a histogram formed from the change in altitude between consecutive heel strikes identified in the movement measurements, and the histogram also includes vertical bars at around +0.18 m and -0.18 m corresponding to the riser height of a step/stair. It can be seen that the histogram is generally centred on the riser heights of 0 m to -0.18 m, and so this movement can be classified as the subject traversing (descending) stairs.

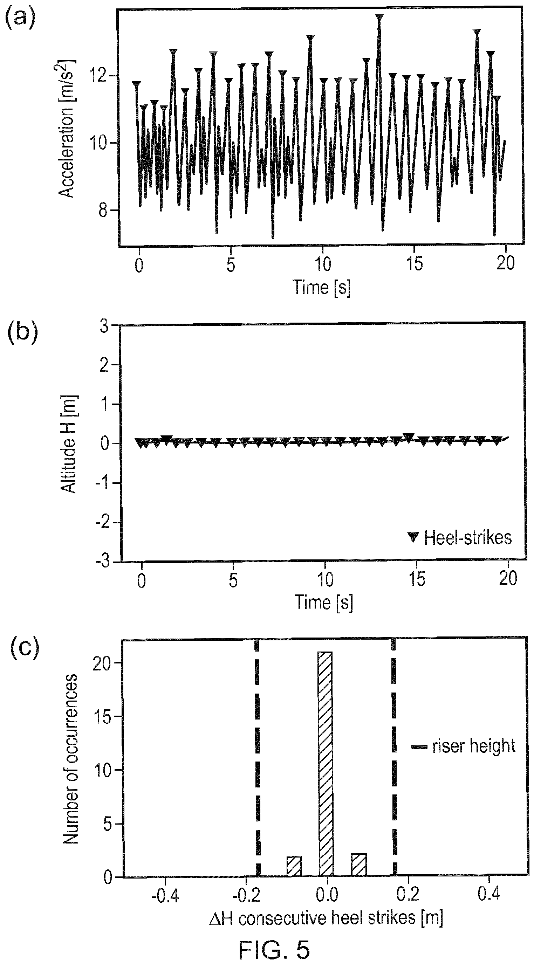

In Fig. 5, the measurements are for a subject that is walking on a flat surface. Thus, Fig. 5(a) shows an exemplary acceleration signal for the 15-second time period, with a series of identified heel-strikes, which correspond to alternating heel strikes by the left foot and the right foot, marked by inverted triangles. Fig. 5(b) shows an exemplary altitude signal for the same 15-second time period represented in Fig. 5(a). This altitude signal is derived from the air pressure signal using a conventional relationship between air pressure and altitude with the initial altitude (i.e. at the start of the 15-second time period) being set to 0. It can be seen that the altitude is relatively constant for the entire 15-second period. Fig. 5(b) shows the parts of the air pressure signal selected according to step 107 for each of the identified heel strikes. Fig. 5(c) shows a histogram formed from the change in altitude between consecutive heel strikes identified in the movement measurements, and the histogram also includes vertical bars at around +0.18 m and -0.18 m corresponding to the riser height of a step/stair. It can be seen that the histogram is (almost completely) centred on a change in altitude of 0 m, and so this movement would not be classified as the subject traversing stairs.

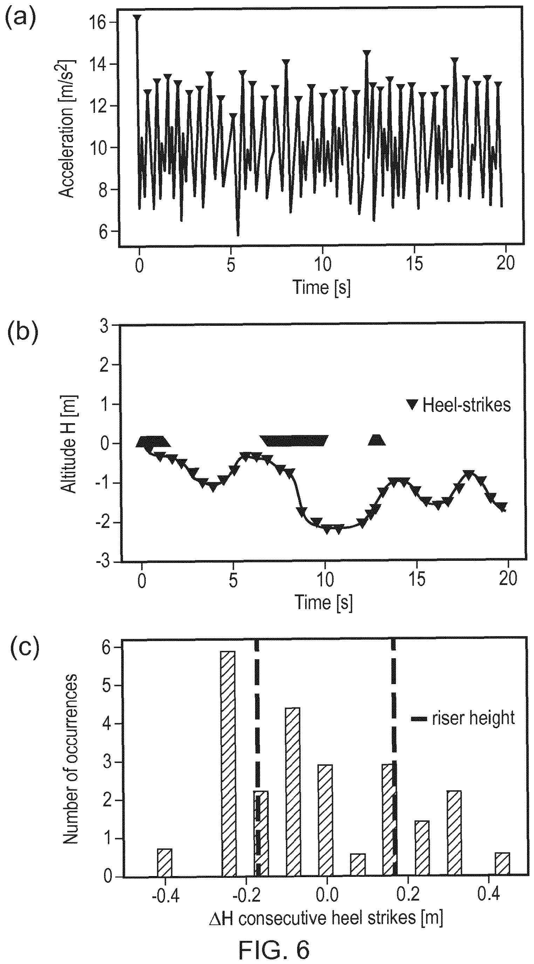

In Fig. 6, the measurements are for a first subject that is walking on an uneven surface and/or where measurement artefacts are present in the air pressure measurements (e.g. due to a change in the environmental conditions). Thus, Fig. 6(a) shows an exemplary acceleration signal for the 15-second time period, with a series of identified heel-strikes, which correspond to alternating heel strikes by the left foot and the right foot, marked by inverted triangles. Fig. 6(b) shows an exemplary altitude signal for the same 15-second time period represented in Fig. 6(a). This altitude signal is derived from the air pressure signal using a conventional relationship between air pressure and altitude with the initial altitude (i.e. at the start of the 15-second time period) being set to 0. It can be seen that the altitude trend over the 15-second period is a decrease, although there are periods of increasing and decreasing altitude. Fig. 6(b) shows the parts of the air pressure signal selected according to step 107 for each of the identified heel strikes. Fig. 6(c) shows a histogram formed from the change in altitude between consecutive heel strikes identified in the movement measurements, and the histogram also includes vertical bars at around +0.18 m and -0.18 m corresponding to the riser height of a step/stair. It can be seen that the histogram is not centred around any particular change in altitude, and although the histogram 'leans' toward an altitude change consistent with a step/stair descent, there are also a relatively significant number of altitude changes consistent with a step/stair ascent. Therefore this movement would not be classified as the first subject traversing stairs.

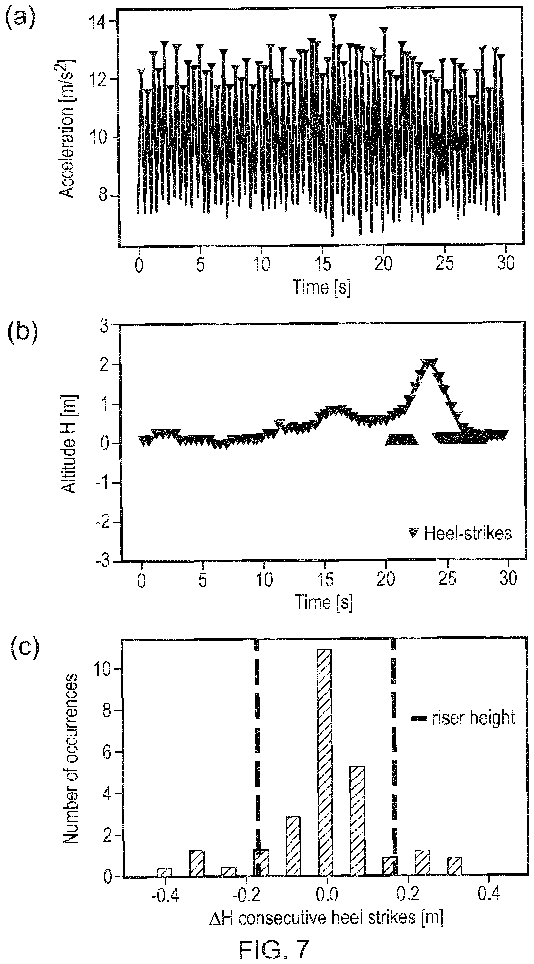

In Fig. 7, the measurements are for a second subject that is walking on an uneven surface and/or where measurement artefacts are present in the air pressure measurements (e.g. due to a change in the environmental conditions). Thus, Fig. 7(a) shows an exemplary acceleration signal for the 15-second time period, with a series of identified heel-strikes, which correspond to alternating heel strikes by the left foot and the right foot, marked by inverted triangles. Fig. 6(b) shows an exemplary altitude signal for the same 15-second time period represented in Fig. 6(a). This altitude signal is derived from the air pressure signal using a conventional relationship between air pressure and altitude with the initial altitude (i.e. at the start of the 15-second time period) being set to 0. It can be seen that the altitude trend over the 15-second period is constant, although there is a period of time where the altitude appears to increase relatively sharply (i.e. an increase of around 1.5 m in 3 seconds) followed by an equally sharp decrease. Fig. 7(b) shows the parts of the air pressure signal selected according to step 107 for each of the identified heel strikes. Fig. 7(c) shows a histogram formed from the change in altitude between consecutive heel strikes identified in the movement measurements, and the histogram also includes vertical bars at around +0.18 m and -0.18 m corresponding to the riser height of a step/stair. It can be seen that the histogram is largely centred on a 0 m change in altitude, and therefore this movement would not be classified as the subject traversing stairs.

In some embodiments, to improve the power consumption or computation requirements of the techniques described herein, one of the movement sensor 6 and the air pressure sensor 8 can be deactivated or powered off while the subject is not moving or otherwise likely to be traversing stairs. For example, the air pressure sensor 8 can be deactivated or powered off while the movement sensor 6 does not detect any movement by the subject, or where the some characteristic of the movement measurements does not meet a criterion (e.g. an amount of movement or magnitude of the movement is below a threshold amount). If the characteristic meets the criterion (e.g. the amount of movement or magnitude of the movement is above the threshold amount), for example indicating that the subject is moving, the air pressure sensor 8 can be activated or otherwise powered on so that it measures the air pressure at the device 4. Otherwise, the air pressure sensor 8 can be deactivated (if not already deactivated) or powered off (if not already powered off). In this way, the movement sensor 6 and the air pressure sensor 8 will be measuring the movements and air pressure respectively at a time where the subject may be traversing stairs.

Thus, the method in Fig. 2 can further comprise obtaining one of a movement signal from the movement sensor 6 for a second time period (which is before the first time period) and an air pressure signal from the air pressure sensor 8 for the second time period while the other one of the movement sensor 6 and air pressure sensor 8 is not active, processing the obtained signal to determine a value of a characteristic of the one of the signal (e.g. a variance of the signal, a maximum amplitude of the signal, a minimum amplitude of the signal, a difference between a maximum amplitude and a minimum amplitude of the signal, etc.), and activating the other one of the movement sensor 6 and air pressure sensor 8 if the determined value meets a criterion (e.g. the determined value is above a threshold).

Furthermore, while both the movement sensor 6 and the air pressure sensor 8 are active or powered on, the movement signal and/or air pressure signal can be analysed to determine a value for the characteristic, and if the determined value does not meet the criterion, one of the movement sensor 6 and air pressure sensor 8 are deactivated.

In some embodiments, the method in Fig. 2 can further comprise outputting an indication of whether the subject has traversed stairs, based on the result of step 111. The output can be provided via the user interface 18, for example as a visual and/or audible output, and/or it can be communicated as a signal to another apparatus or computer via the interface circuitry 16.

In some embodiments, the result of step 111 and the movement measurements associated with traversing stairs can be used to determine a fall risk for the subject and/or an indication of the mobility of the subject. That is, the result of step 111 and the movement measurements (and optionally the air pressure measurements) associated with the subject traversing stairs, can be analysed using a fall risk estimation algorithm and/or a mobility estimation algorithm to determine a measure of the fall risk and/or measure of the mobility of the subject. Those skilled in the art will be aware of various fall risk estimation algorithms and/or mobility estimation algorithms that can be used to estimate a fall risk and/or estimate mobility from an indication of the subject having traversed stairs and the associated movement measurements, and so further details are not provided herein.

Therefore there is provided an improved technique for the analysis of movement measurements and air pressure measurements to determine if a subject has traversed stairs.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method for analysing movement of a subject, the method comprising:
obtaining, from a movement sensor in a device that is carried or worn by the subject, a movement signal representing movement of the subject during at least a first time period;
obtaining, from an air pressure sensor in the device, an air pressure signal representing air pressure at the air pressure sensor during at least the first time period;
processing the movement signal to identify a plurality of occurrences of a gait phase corresponding to the subject walking;
for each of the identified occurrences of the gait phase, selecting a respective part of the air pressure signal corresponding in time to said occurrence of the gait phase;
for a plurality of pairs of identified occurrences of the gait phase, determining a change in altitude of the subject between the identified occurrences of the gait phase in each pair from the respective selected parts of the air pressure signal; and
determining if the subject has traversed stairs from the determined changes in altitude for the pairs of identified occurrences of the gait phase.

2. A method as claimed in claim 1, wherein the step of determining if the subject has traversed stairs from the determined changes in altitude for the pairs of identified occurrences of the gait phase comprises:
determining one or more statistics values from the determined changes in altitude for the pairs of identified occurrences of the gait phase; and
determining if the subject has traversed stairs based on the determined one or more statistics values.

3. A method as claimed in claim 2, wherein the step of determining if the subject has traversed stairs based on the determined one or more statistics values comprises comparing the one or more statistics values to one or more normal values and/or historical values for the subject when traversing stairs.

4. A method as claimed in claim 2 or 3, wherein the step of determining if the subject has traversed stairs based on the determined one or more statistics values comprises comparing the one or more statistics values to one or more standard values for a set of stairs.

5. A method as claimed in any of claims 2-4, wherein the one or more statistics comprises an average change in altitude per gait phase occurrence, an average change in altitude per gait phase occurrence for occurrences of the gait phase occurring in a predetermined time period, or a measure of variability of the determined changes in altitude for the pairs of identified occurrences of the gait phase.

6. A method as claimed in any of claims 2-5, wherein the step of determining one or more statistics values comprises forming a histogram from the determined changes in altitude for the pairs of identified occurrences of the gait phase; and
wherein the step of determining if the subject has traversed stairs comprises determining if the subject has traversed stairs based on the histogram.

7. A method as claimed in any of claims 1-6, wherein the step of selecting the respective part of the air pressure signal corresponding in time to said occurrence of the gait phase comprises:
selecting a single measurement sample from the air pressure signal corresponding in time to the identified occurrence of the gait phase; or
selecting a plurality of measurement samples from the air pressure signal corresponding in time to the identified occurrence of the gait phase.

8. A method as claimed in any of claims 1-7, wherein, prior to the first time period, the method further comprises the steps of:
obtaining one of a movement signal from the movement sensor for a second time period and an air pressure signal from the air pressure sensor for a second time period while the other one of the movement sensor and air pressure sensor is not active;
processing the obtained one of the movement signal and the air pressure signal to determine a first value of a characteristic of the one of the movement signal and the air pressure signal; and
if the determined first value meets a criterion, activating the other one of the movement sensor and air pressure sensor.

9. A method as claimed in claim 8, wherein the method further comprises the steps of:
obtaining a further one of the movement signal from the movement sensor for a third time period and an air pressure signal from the air pressure sensor for a third time period;
processing the obtained further one of the movement signal and the air pressure signal to determine a second value of the characteristic; and
if the determined second value does not meet the criterion, deactivating the other one of the movement sensor and air pressure sensor.

10. A computer program product comprising a computer readable medium having computer readable code embodied therein, the computer readable code being configured such that, on execution by a suitable computer or processor, the computer or processor is caused to perform the method of any of claims 1-9.

11. An apparatus for analysing movement of a subject, the apparatus comprising a processing unit configured to:
obtain, from a movement sensor in a device that is carried or worn by the subject, a movement signal representing movement of the subject during at least a first time period;
obtain, from an air pressure sensor in the device, an air pressure signal representing air pressure at the air pressure sensor during at least the first time period;
process the movement signal to identify a plurality of occurrences of a gait phase corresponding to the subject walking;
for each of the identified occurrences of the gait phase, select a respective part of the air pressure signal corresponding in time to said occurrence of the gait phase;
for a plurality of pairs of identified occurrences of the gait phase, determine a change in altitude of the subject between the identified occurrences of the gait phase in each pair from the respective selected parts of the air pressure signal; and
determine if the subject has traversed stairs from the determined changes in altitude for the pairs of identified occurrences of the gait phase.

12. An apparatus as claimed in claim 11, wherein the processing unit is configured to determine if the subject has traversed stairs from the determined changes in altitude for the pairs of identified occurrences of the gait phase by:
determining one or more statistics values from the determined changes in altitude for the pairs of identified occurrences of the gait phase; and
determining if the subject has traversed stairs based on the determined one or more statistics values.

13. An apparatus as claimed in claim 11 or 12, wherein the processing unit is configured to determine one or more statistics values by forming a histogram from the determined changes in altitude for the pairs of identified occurrences of the gait phase; and wherein the processing unit is configured to determine if the subject has traversed stairs based on the histogram.

14. An apparatus as claimed in any of claims 11-13, wherein the processing unit is configured to select the respective part of the air pressure signal corresponding in time to said occurrence of the gait phase by:
selecting a single measurement sample from the air pressure signal corresponding in time to the identified occurrence of the gait phase; or
selecting a plurality of measurement samples from the air pressure signal corresponding in time to the identified occurrence of the gait phase.

15. A system for analysing movement of a subject, the system comprising:
a device that is to be carried or worn by the subject, the device comprising a movement sensor for measuring movement of the subject and outputting a movement signal representing movement of the subject and an air pressure sensor for measuring air pressure and outputting an air pressure signal representing air pressure at the air pressure sensor; and
an apparatus as claimed in any of claims 11-14.
